# FASCICULE DE BREVET EUROPEEN

(11) **EP 0 499 516 B1**
(45) Date de publication et mention de la délivrance du brevet: **25.01.1995**
(21) Numéro de dépôt: 92400330.4
(22) Date de dépôt: 07.02.1992
(51) Int. Cl.: C07C 19/08, C07C 17/00

(54) **Synthèse d'hydrogéno-perfluoroalcanes**
Verfahren zur Synthese von Hydrogenoperfluoralkanen
Process for the synthesis of hydrogeno-perfluoroalkanes

(30) Priorité: 12.02.1991 FR 9101617
(43) Date de publication de la demande: 19.08.1992
(73) Titulaire: ELF ATOCHEM S.A., 92800 Puteaux (FR)
(72) Inventeur: Durual, Pierre, F-69390 Vernaison (FR); Lantz, André, F-69390 Vernaison (FR)
(74) Mandataire: Leboulenger, Jean

(56) Documents cités:
- EP-A- 0 308 923
- FR-A- 2 116 524
- JOURNAL OF THE CHEMICAL SOCIETY. 1951, LETCHWORTH GB pages 60 - 64; J.BANUS ET AL: 'The Heterolytic Fission of the Carbon-Iodine Bond in Trifluoroiodomethane'

## Description

La présente invention concerne le domaine des hydrocarbures aliphatiques fluorés et a plus particulièrement pour objet la préparation des hydrogéno-perfluoroalcanes R_{F}H, R_{F} désignant un radical perfluoroalkyle linéaire ou ramifié qui peut comporter de 2 à 20 atomes de carbone, de préférence 2 à 10.

Les hydrogéno-perfluoroalcanes sont des composés connus qui peuvent être utilisés par exemple comme fluides inertes dans le domaine de l'électronique, comme fluides caloporteurs ou comme produits intermédiaires pour la synthèse d'autres composés fluorés, par exemple pour la préparation des bromures de perfluoroalkyle R_{F}Br dont certains (notamment C₈F₁₇Br) ont trouvé une application en médecine comme radiopaques (agents de contraste aux rayons X) ou comme transporteurs d'oxygène dans les substituts du sang.

Parmi les nombreuses méthodes connues pour préparer les hydrogéno-perfluoroalcanes, les plus attractives, car partant des iodures de perfluoroalkyle R_{F}I qui sont des produits industriels, consistent à faire réagir un composé R_{F}I avec la potasse alcoolique à une température de 100 à 130°C (voir J. BANUS et al, J. Chem. Soc. 1951, 60 et surtout R.N. HASZELDINE, J. Chem. Soc. 1953, 3761-8) ou avec l'hydrogène sous pression en présence de nickel de Raney (R.N. HASZELDINE, ibid. et brevet FR 2 116 524).

Bien qu'elle apparaisse fournir les meilleurs rendements (> 80 %), cette dernière méthode présente l'inconvénient, d'une part, d'opérer en autoclave sous une pression de plusieurs dizaines de bars d'hydrogène et, d'autre part, de ne pas conduire à une sélectivité totale puisqu'il se forme des produits de duplication R_{F}-R_{F} qui polluent les hydrogéno-perfluoroalcanes obtenus.

La première méthode (traitement du R_{F}I par la potasse alcoolique) ne présente pas ces inconvénients, mais conduit malheureusement à un rendement en composé R_{F}H (27 à 51 %) d'autant plus faible que le nombre d'atomes de carbone du radical R_{F} est plus élevé (51 % pour C₃F₇H et 27 % pour C₈F₁₇H). On observe en outre la formation de goudrons qui empêchent la filtration du mélange réactionnel et rendent impossible la récupération de l'iodure de potassium sous-produit. Il en est de même lorsqu'on remplace l'éthanol par l'isopropanol utilisé dans le brevet US 4 618 731 relatif à la purification des perfluoroalkyl-2 éthanols contenant des composés R_{F}CH₂CH₂I et R_{F}I comme impuretés.

Il a maintenant été trouvé de façon tout-à-fait inattendue que le méthanol ne conduit pas à la formation de goudrons et permet d'obtenir non seulement une conversion quasi-totale du R_{F}I de départ, mais aussi un R_{F}H d'excellente pureté. L'emploi du méthanol permet en outre de récupérer la majeure partie du iodure alcalin sous-produit.

L'invention a donc pour objet un procédé de préparation d'un hydrogéno-perfluoroalcane R_{F}H par réaction du iodure de perfluoroalkyle R_{F}I correspondant avec un hydroxyde alcalin, caractérisé en ce qu'on opère dans le méthanol.

Comme hydroxyde alcalin, on préfère utiliser l'hydroxyde de potassium car l'iodure de potassium sous-produit, facilement récupérable avec une excellente pureté grâce à l'emploi du méthanol, est un produit demandé sur le marché. On ne sortirait cependant pas du cadre de la présente invention en mettant en oeuvre l'hydroxyde d'un autre métal alcalin tel que, par exemple, l'hydroxyde de sodium. La quantité d'hydroxyde alcalin à utiliser par mole de R_{F}I est de 1 à 5 moles ; elle est de préférence comprise entre 1,5 et 3 moles.

La quantité de méthanol à utiliser (0,25 à 3 litres, de préférence 0,5 à 1,5 litre, par kilogramme de R_{F}I engagé) doit être suffisante pour une solubilisation partielle des réactifs de façon à assurer un contact suffisant pour réaliser la réaction.

Le procédé selon l'invention peut être réalisé en engageant dès le départ la totalité des réactifs (R_{F}I, hydroxyde alcalin, méthanol). Cependant, pour éviter d'éventuels emballements dus à l'exothermicité de la réaction, il est préférable d'ajouter de façon progressive (par exemple en 1 à 20 heures et de préférence en 2 à 8 heures) soit de R_{F}I dans la solution méthanolique d'hydroxyde alcalin, soit cette dernière solution dans une solution méthanolique de R_{F}I.

La réaction est effectuée à une température allant de 10 à 66°C, la température optimale dépendant du R_{F}H considéré.

L'hydrogéno-perfluoroalcane formé peut être récupéré par distillation du mélange réactionnel. Cette distillation fournit soit le R_{F}H lui-même (cas des composés en C₂ à C₄), soit un azéotrope R_{F}H/méthanol (cas des composés lourds comme C₆F₁₃H ou C₈F₁₇H) ; un lavage à l'eau de l'azéotrope permet d'isoler de R_{F}H sous une forme extrêmement pure.

Il peut également être avantageux de distiller en continu le R_{F}H ou l'azéotrope R_{F}H/méthanol, en particulier dans le cas où l'on effectue la réaction de façon progressive en ajoutant de R_{F}I dans la solution méthanolique d'hydroxyde alcalin ou en ajoutant la solution méthanolique d'hydroxyde alcalin dans une solution méthanolique de R_{F}I.

Les exemples suivants illustrent l'invention, sans la limiter. Les pourcentages sont exprimés en poids. 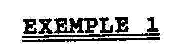
Dans un réacteur de 2 litres muni d'une agitation, d'un thermomètre, d'une ampoule de coulée et surmonté d'une colonne à distiller à garnissage verre suivie d'un condenseur, on charge 1 litre de méthanol et 198 g de potasse à 85 % (3 moles de KOH), puis on introduit sous agitation, en 2 heures, 1092 g (2 moles) d'iodure de perfluorooctyle. La réaction est exothermique.

Par chauffage, on distille à 62-63°C un azéotrope C₈F₁₇H/méthanol (59/41) qu'on recueille dans 500 g d'eau pendant 5 heures. Par décantation, on sépare une phase inférieure (825 g) constituée de C₈F₁₇H à 99,4 % de pureté. Rendement : 97,6 %.

Par filtration du pied du réacteur, on récupère 279 g d'un solide constitué d'iodure de potassium à 97 % de pureté (rendement : 84 %). 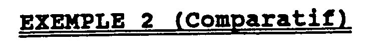
On opère comme à l'exemple 1, mais en remplaçant le méthanol par la même quantité d'éthanol et en utilisant seulement 132 g de potasse à 85 % (2 moles de KOH).

Après 3 heures de distillation à 78°C en tête (éthanol), on constate que la réaction est bloquée. On a recueilli 499 g d'un mélange composé de 84,4 % de C₈F₁₇H, 12,8 % d'éthanol et 1,3 % de C₈F₁₇I, soit un rendement de 50 %.

Le mélange réactionnel resté dans le réacteur est tout noir. On lui ajoute 132 g de potasse à 85 %, puis on reprend la distillation pendant 2 heures. La réaction est encore bloquée et on a recueilli 258 g de produit contenant 81 % de C₈F₁₇H, 16,6 % d'éthanol et 1,6 % de C₈F₁₇I, ce qui porte le rendement global à 75 %. Le contenu du réacteur se présente sous forme d'une masse noire goudronneuse, infiltrable. 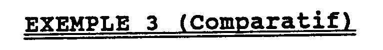
On reproduit l'exemple 1, mais en remplaçant le méthanol par l'isopropanol. Par distillation jusqu'à 74°C, on recueille 102 g d'une phase homogène comprenant 20,6 % de C₈F₁₇H, 21,8 % d'isopropanol et 57,1 % d'acétone.

En poursuivant la distillation à 74-76°C, on recueille un produit qui décante en deux phases, la phase inférieure (250 g) comprenant 67,4 % de C₈F₁₇H, 24,1 % d'isopropanol et 8,2 % d'acétone. Le rendement global en C₈F₁₇H est de 22,6 %.

En fin de distillation, le contenu du réacteur se solidifie en une masse noire qui bloque l'agitation, ce qui conduit à arrêter l'essai. 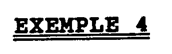
On opère comme à l'exemple 1 avec 400 ml de méthanol, 107 g de potasse à 85 % (1,62 mole de KOH) et 446 g (1 mole) d'iodure de perfluorohexyle.

Après distillation à 52,5-53°C de l'azéotrope C₆F₁₃H/méthanol (89/11), on poursuit la distillation jusqu'à 60°C en tête, puis le distillat (332 g) est versé dans 200 ml d'eau. Après décantation, la phase inférieure est lavée avec 200 ml d'eau. On obtient ainsi 289 g de C₆F₁₃H à 99,7 % de pureté. Rendement : 90 %. 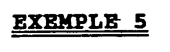
Dans un réacteur de 4 litres, équipé d'une agitation, d'un thermomètre et d'une colonne de distillation à garnissage en verre refroidie à -20°C, on charge 1,5 litre de méthanol et 296,5 g de potasse à 85 % (4,5 moles de KOH). On porte le mélange à 35°C, puis on introduit en 4 heures 1038 g (3 moles) d'iodure de perfluorobutyle tout en maintenant la température du mélange réactionnel à 40-45°C.

Pendant l'introduction du C₄F₉I, on distille en tête (T = 16°C) de nonafluorobutane C₄F₉H formé. Ce composé qui ne forme pas d'azéotrope avec le méthanol est recueilli dans une ampoule refroidie par un bain de glace.

Après la fin de l'introduction du C₄F₉I, on poursuit la distillation jusqu'à 62°C en tête de façon à récupérer la totalité du C₄F₉H formé. On obtient ainsi 647 g de C₄F₉H à 99,3 % de pureté (0,1 % de perfluorooctane, 0,2 % de C₄F₉I entraîné et 0,3 % de méthanol). Rendement : 97,3 %. 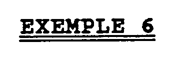
On utilise deux réacteurs de 1 litre, disposés en série et munis de moyens d'agitation et de chauffage. Dans le premier réacteur on charge une solution de 99 g de potasse à 85 % (1,5 mole de KOH) dans 0,5 litre de méthanol et dans le second réacteur une solution de 20 g de potasse à 85 % (0,3 mole de KOH) dans 0,5 litre de méthanol.

On chauffe le premier réacteur à 30°C, puis on y introduit en 7 heures 246 g (1 mole) d'iodure de pentafluoroéthyle tout en maintenant sous agitation à 30-35°C.

Les gaz passent dans le second réacteur agité et maintenu à 20-25°C, puis dans un flacon laveur à eau pour éliminer le méthanol entraîné. Ils sont ensuite séchés sur CaCl₂ avant d'être recueillis dans un piège refroidi par carboglace.

L'introduction de C₂F₅I terminée, on porte à reflux le contenu du premier réacteur, puis celui du second réacteur pour récupérer tout de C₂F₅H dissous.

On obtient ainsi 114 g de pentafluoroéthane contenant 2 % de C₂F₅I résiduel. Rendement en C₂F₅H : 93,1 %. 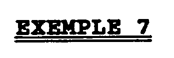
Dans un réacteur de 2 litres, muni d'une agitation, d'un thermomètre, d'une ampoule de coulée et surmonté d'un premier élément de colonne à distiller à garnissage verre refroidie à l'eau, puis d'un deuxième élément identique refroidi à -25°C, on charge 200 ml de méthanol et 1184 g (4 moles) d'iodure de perfluoro-isopropyle.

On chauffe le réacteur à 40°C, puis on introduit en 2 heures et demie, en maintenant à 40-45°C, une solution de 420 g de potasse à 85 % (6,4 moles de KOH) dans 1,2 litre de méthanol. Pendant l'introduction de cette solution, le C₃F₇H distille et on le recueille dans un piège refroidi par carboglace.

Après introduction de toute la solution de potasse méthanolique, on chauffe le contenu du réacteur jusqu'à 66°C pour récupérer tout de C₃F₇H formé. On recueille ainsi 667,5 g de C₃F₇H à 99,5 % de pureté, soit un rendement de 97,7 %.

## Revendications

1. Procédé de préparation d'un hydrogéno-perfluoroalcane R_{F}H, R_{F} désignant un radical perfluoroalkyle linéaire ou ramifié comportant de 2 à 20 atomes de carbone, par réaction du iodure de perfluoroalkyle R_{F}I correspondant avec un hydroxyde alcalin à raison de 1 à 5 moles d'hydroxyde alcalin par mole de R_{F}I, caractérisé en ce qu'on opère dans le méthanol à une température comprise entre 10 et 66°C, la quantité de méthanol étant comprise entre 0,25 et 3 litres par kg de R_{F}I.

2. Procédé selon la revendication 1, dans lequel l'hydroxyde alcalin est l'hydroxyde de potassium.

3. Procédé selon la revendication 1 ou 2, dans lequel la quantité d'hydroxyde alcalin, utilisée par mole de R_{F}I, est comprise entre 1,5 et 3 moles.

4. Procédé selon l'une des revendications 1 à 3, dans lequel la quantité de méthanol, utilisée par kg de R_{F}I est comprise entre 0,5 et 1,5 litre.

5. Procédé selon l'une des revendications 1 à 4, dans lequel le R_{F}I est introduit dans une solution méthanolique d'hydroxyde alcalin de façon progressive, de préférence en 2 à 8 heures.

6. Procédé selon l'une des revendications 1 à 4, dans lequel on introduit une solution méthanolique d'hydroxyde alcalin dans une solution méthanolique de R_{F}I.

7. Procédé selon l'une des revendications 1 à 6, dans lequel on opère à une température suffisante pour distiller le R_{F}H formé ou son azéotrope avec le méthanol.

8. Procédé selon la revendication 7, caractérisé en ce qu'on utilise l'hydroxyde de potassium et récupère l'iodure de potassium sous-produit par filtration du mélange réactionnel résiduaire.

9. Application du procédé selon l'une des revendications 1 à 8, à la synthèse des hydrogéno-perfluoroalcanes en C₂ à C₁₀, en particulier à celle de C₈F₁₇H.

## Claims

1. Process for the preparation of a hydrogenperfluoroalkane R_{F}H, R_{F} denoting a linear or branched perfluoroalkyl radical containing from 2 to 20 carbon atoms, by reaction of the corresponding perfluoroalkyl iodide R_{F}I with an alkali metal hydroxide in a proportion of 1 to 5 mol of alkali metal hydroxide per mole of R_{F}I, characterized in that the reaction is carried out in methanol at a temperature between 10 and 66°C, the amount of methanol being between 0.25 and 3 litres per kg of R_{F}I.

2. Process according to Claim 1, in which the alkali metal hydroxide is potassium hydroxide.

3. Process according to Claim 1 or 2, in which the amount of alkali metal hydroxide used per node of R_{F}I is between 1.5 and 3 mol.

4. Process according to one of Claims 1 to 3, in which the amount of methanol used per kg of R_{F}I is between 0.5 and 1.5 litres.

5. Process according to one of Claims 1 to 4, in which the R_{F}I is progressively introduced into a methanolic alkali metal hydroxide solution, preferably over 2 to 8 hours.

6. Process according to one of Claims 1 to 4, in which a methanolic alkali metal hydroxide solution is introduced into a methanolic R_{F}I solution.

7. Process according to one of Claims 1 to 6, in which the reaction is carried out at a temperature sufficient to distil the R_{F}H formed or its azeotrope with methanol.

8. Process according to Claim 7, characterized in that potassium hydroxide is used and the potassium iodide by-product is recovered by filtration of the residual reaction mixture.

9. Application of the process according to one of Claims 1 to 8 to the synthesis of C₂ to C₁₀ hydrogen-perfluoroalkanes, in particular to that of C₈F₁₇H.

## Patentansprüche

1. Verfahren zur Herstellung eines Hydrogeno-Perfluoralkans R_{F}H, wobei R_{F} einen linearen oder verzweigten Perfluoralkylrest mit 2 bis 20 Kohlenstoffatomen bezeichnet, durch Reaktion des entsprechenden Perfluoralkyljodids R_{F}I mit einem Alkalihydroxid im Verhältnis von 1 bis 5 Mol Alkalihydroxid pro Mol R_{F}I, dadurch gekennzeichnet, daß die Reaktion im Methanol bei einer Temperatur zwischen 10 und 66 °C durchgeführt wird, und die Menge an Methanol zwischen 0,25 und 3 l/kg R_{F}I beträgt.

2. Verfahren nach Anspruch 1, in dem das Alkalihydroxid Kaliumhydroxid ist.

3. Verfahren nach Anspruch 1 oder 2, in dem die Menge an Alkalihydroxid, die pro Mol R_{F}I verwendet wird, zwischen 1,5 und 3 Mol liegt.

4. Verfahren nach einem der Ansprüche 1 bis 3, in dem die Menge an Methanol, die pro kg R_{F}I verwendet wird, zwischen 0,5 und 1,5 l liegt.

5. Verfahren nach einem der Ansprüche 1 bis 4, in dem das R_{F}I in eine methanolische Alkalihydroxidlösung nach und nach hinzugegeben wird, vorzugsweise innerhalb von 2 bis 8 Stunden.

6. Verfahren nach einem der Ansprüche 1 bis 4, in dem man eine methanolische Alkalihydroxidlösung in eine methanolische Lösung von R_{F}I gibt.

7. Verfahren nach einem der Ansprüche 1 bis 6, in den, man bei einer genügend hohen Temperatur arbeitet, um das gebildete R_{F}H oder sein Azeotrop mit Methanol zu destillieren.

8. Verfahren nach Anspruch 7, dadurch gekennzeichnet, daß man Kaliumhydroxid verwendet und daß man das als Nebenprodukt gebildete Kaliumjodid durch Filtration der restlichen Reaktionsmischung zurückgewinnt.

9. Verwendung des Verfahrens nach einem der Ansprüche 1 bis 8 zur Herstellung von C₂-C₁₀-Hydrogeno-Perfluoralkanen, insbesondere zur Herstellung von C₈F₁₇H.
